(19) Europäisches Patentamt / European Patent Office / Office européen des brevets

(11) **EP 4 501 243 A1**

(12) **EUROPEAN PATENT APPLICATION**
published in accordance with Art. 153(4) EPC

(43) Date of publication:
05.02.2025 Bulletin 2025/06

(21) Application number: 23774001.4

(22) Date of filing: 24.03.2023

(51) International Patent Classification (IPC):
*A61B 8/00* $^{(2006.01)}$    *A61B 34/10* $^{(2016.01)}$

(86) International application number:
PCT/CN2023/083618

(87) International publication number:
WO 2023/179756 (28.09.2023 Gazette 2023/39)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 24.03.2022 CN 202210291564

(71) Applicant: Healinno (Beijing) Medical Technology
Co., Ltd.
Beijing 100176 (CN)

(72) Inventors:
• SHI, Yilun
Beijing 100176 (CN)

• ZHAO, Jing
Beijing 100176 (CN)
• MA, Tao
Beijing 100176 (CN)
• CHEN, Wenbo
Beijing 100176 (CN)
• SHI, Ce
Beijing 100176 (CN)

(74) Representative: Zaboliene, Reda
Metida
Business center Vertas
Gyneju str. 16
01109 Vilnius (LT)

(54) **BIPLANE ULTRASOUND IMAGE PLANNING METHOD AND APPARATUS**

(57)    The present application discloses a biplane ultrasound image planning method, which solves the problem of large path planning errors in existing methods. The method comprises: calibrating a position relationship between a marker and a cross-sectional image to obtain a first conversion matrix, the first conversion matrix being a matrix converted from a coordinate system of the cross-sectional image to a coordinate system of the marker; calibrating a position relationship between the marker and a sagittal image to obtain a second conversion matrix, the second conversion matrix being a matrix converted from a coordinate system of the sagittal image to the coordinate system of the marker; and calculating a third or fourth conversion matrix by means of a matrix conversion relationship, the third conversion matrix being a matrix converted from the coordinate system of the cross-sectional image to the coordinate system of the sagittal image, and the fourth matrix being a matrix converted from the coordinate system of the sagittal image to the coordinate system of the cross-sectional image.

Calibrate a position relationship between a first ultrasonic probe and a cross-sectional image to obtain a first conversion matrix — 101

Calibrate a position relationship between the first ultrasonic probe and a sagittal image to obtain a second conversion matrix — 102

Calculate a third and/or fourth index conversion matrix by using a matrix conversion relationship, and display a target position displayed in any one of the coordinate system of the cross-sectional image and the coordinate system of the sagittal image in the other coordinate system in a follow-up manner — 103

FIG. 1(a)

EP 4 501 243 A1

Description

## CROSS-REFERENCE TO RELATED APPLICATIONS

The present application claims priority to Chinese Patent Application No. 202210291564.X, filed on March 24, 2022. The present application includes the entire contents of the application with reference to the application..

## TECHINCAL FIELD

The present application relates to the technical field of medical electronics, and in particular to a biplanar ultrasound image planning method and apparatus.

## BACKGROUND

A biplanar ultrasonic probe can be commonly used in urology and anorectal surgery. Transrectal ultrasound (TRUS) examination includes a high-resolution two-dimensional image display technology and a color Doppler technology. Transrectal biplanar probe examination has high ultrasonic frequency and can obtain high-resolution images in two directions, thereby clearly displaying the prostate blood flow distribution characteristic and echo intensity. A doctor needs to obtain ultrasound images in two different directions (cross section and sagittal plane) through a transrectal biplanar ultrasonic probe during surgery. The doctor can perform spatial position identification and manual labeling by respectively reading the cross-sectional and sagittal images, or manual input parameters, and information input by the doctor is converted into cutting path planning during surgery. The doctor needs to repeatedly move the ultrasonic probe to observe the target position when manually using the biplanar ultrasound. The spatial position relationship between two planes is determined generally by identifying a structural key area in the image, which is time-consuming, laborious and poor in accuracy and stability.

## SUMMARY

The present application provides a biplanar ultrasound image planning method and apparatus, which solve the problem of large path errors in existing methods, and are particularly suitable for surgical scenarios such as urological intervention, particle implantation therapy and tissue resection.

To solve the above problem, the present application is implemented as follows.

According to a first aspect, the present application provides a biplanar ultrasound image planning method, which is a biplanar ultrasound image planning method captured by an ultrasonic probe, and includes the following steps:

calibrating a position relationship between a marker and a cross-sectional image to obtain a first conversion matrix, the first conversion matrix being a matrix converted from a coordinate system of the cross-sectional image to a coordinate system of the marker;

calibrating a position relationship between the marker and a sagittal image to obtain a second conversion matrix, the second conversion matrix being a matrix converted from a coordinate system of the sagittal image to a coordinate system of the marker;

calculating a third conversion matrix and or a fourth conversion matrix based on the first conversion matrix and the second conversion matrix, the third conversion matrix being a matrix converted from the coordinate system of the cross-sectional image to the coordinate system of the sagittal image, and the fourth matrix being a matrix converted from the coordinate system of the sagittal image to the coordinate system of the cross-sectional image; and

through the third conversion matrix and or the fourth conversion matrix, displaying a target position displayed in any of the coordinate system of the cross-sectional image and the coordinate system of the sagittal image in the other coordinate system in real time and in a follow-up manner.

Preferably, the marker is a biplanar ultrasonic probe.

Preferably, the method further includes:
the marker is either a convex array probe for photographing the cross-sectional image or a linear array probe for photographing the sagittal image.

Preferably, the step of displaying in the other coordinate system further includes:

establishing a first index relationship table or a second index relationship table in the movement process of the ultrasonic probe, the first index relationship table being an index relationship table of a physical position of the ultrasonic probe and a cross-sectional position, and the second index relationship table being an index relationship table of storage position information of the cross-sectional image and the cross-sectional position; and

displaying a target position and a planned position in the cross-sectional image in a follow-up manner by means of the first index relationship table or the second index relationship table and according to a target position and a planned position in the sagittal image.

Preferably, the step of displaying in the other coordinate system further includes:

establishing a third index relationship table or a fourth index relationship table in the movement process of the ultrasonic probe, the third index relationship table being an index relationship table of a physical position of the ultrasonic probe and a sagittal position, and the fourth index relationship table being an index relationship table of storage position information of the sagittal image and the sagittal position; and

displaying a target position and a planned position in the sagittal image in a follow-up manner by means of the third index relationship table or the fourth index relationship table and according to a target position and a planned position in the cross-sectional image.

Preferably, calibrating the position relationship between the marker and the cross-sectional image by a spatial correction method to obtain the first conversion matrix specifically includes:

establishing a fifth conversion matrix $T_{As2st}$ converted from a coordinate system of a positioning needle to a coordinate system of a positioning needle point through positioning needle correction;

obtaining a sixth conversion matrix $T_{As2p}$ converted from the coordinate system of the positioning needle to the coordinate system of the marker through a positioning and tracking system;

calculating a seventh conversion matrix $T_{Ast2p}$ converted from the coordinate system of the positioning needle point to the coordinate system of the marker through the fifth conversion matrix and the sixth conversion matrix;

calculating an eighth conversion matrix $T_{Ast2im}$ converted from the coordinate system of the positioning needle point to the coordinate system of the cross-sectional image according to a position relationship of the positioning needle in the cross-sectional image; and

calculating the first conversion matrix $T_{Aim2p}$ according to the seventh conversion matrix and the eighth conversion matrix.

Preferably, the position relationship between the marker and the sagittal image is calibrated by a spatial correction method to obtain the second conversion matrix,
or inverse matrix operation is performed on the first conversion matrix to obtain the second conversion matrix.
Preferably, the method further includes:
converting a planned position in the coordinate system of the cross-sectional image or the coordinate system of the sagittal image into a motion or energy execution parameter, the motion or energy execution parameter being a parameter for controlling the motion and energy of an execution mechanism.
Preferably, the step of displaying the target position and the planned position in the cross-sectional image in a follow-up manner according to the target position and the planned position in the sagittal image further includes:

calculating a pixel point position, acquiring a target position in the coordinate system of the sagittal image, and calculating a target position in the coordinate system of the cross-sectional image according to the third conversion matrix;

in the first index relationship table, looking up the table to obtain a first cross-sectional position Sx1 and a second cross-sectional position Sx2, obtaining the physical positions of the ultrasonic probe corresponding to Sx1 and Sx2, and obtaining a physical position of a target cross-sectional probe through interpolation, Sx1 and Sx2 being respectively positions of two cross sections closest to Sx, Sx being a cross-sectional position corresponding to si, and si being the target position in the coordinate system of the cross-sectional image;

moving the ultrasonic probe to the physical position of the target cross-sectional probe to obtain a measured cross-sectional image, and obtaining a measured target position in the cross-sectional image in the measured cross-sectional image according to si; and

constructing the planned position in the sagittal image to correspondingly obtain the planned position in the cross-sectional image.

Preferably, the step of displaying the target position and the planned position in the cross-sectional image in a follow-up manner according to the target position and the planned position in the sagittal image further includes:

calculating a pixel point position, acquiring a target position in the coordinate system of the sagittal image, and calculating a target position in the coordinate system of the cross-sectional image according to the third conversion matrix;

in the second index relationship table, looking up the table to obtain a first cross-sectional position Sx1 and a second cross-sectional position Sx2, and obtaining the storage positions of the cross-sectional image corresponding to Sx1 and Sx2, Sx1 and Sx2 being respectively positions of two cross sections closest to Sx, Sx being a cross-sectional position corresponding to si, and si being the target position in the coordinate system of the cross-sectional image;

selecting a cross section closest to Sx from Sx1 and Sx2, displaying a measured cross-sectional image according to the corresponding storage position of the cross-sectional image, and obtaining a measured target position in the cross-sectional image in the measured cross-sectional image according to si; and

constructing the planned position in the sagittal image to correspondingly obtain the planned position in the cross-sectional image.

Preferably, the step of displaying the target position and the planned position in the sagittal image in a follow-up manner according to the target position and the planned position in the cross-sectional image further includes:

calculating a pixel point position, acquiring a target position in the coordinate system of the cross-sectional image, and calculating a target position in the coordinate system of the sagittal image according to the fourth conversion matrix;

in the third index relationship table, looking up the table to obtain a first sagittal position Tx1 and a second sagittal position Tx2, obtaining the physical positions of the ultrasonic probe corresponding to Tx1 and Tx2, and obtaining a physical position of a target sagittal probe through interpolation, Tx1 and Tx2 being positions of two sagittal planes closest to Tx, Tx being a sagittal position corresponding to ti, and ti being the target position in the coordinate system of the sagittal image;

moving the ultrasonic probe to the physical position of the target sagittal probe to obtain a measured sagittal image, and obtaining a measured target position in the sagittal image in the measured sagittal image according to ti; and

constructing the planned position in the cross-sectional image to correspondingly obtain the planned position in the sagittal image.

Preferably, the step of displaying the target position and the planned position in the sagittal image in a follow-up manner according to the target position and the planned position in the cross-sectional image further includes:

calculating a pixel point position, acquiring a target position in the coordinate system of the cross-sectional image, and calculating a target position in the coordinate system of the sagittal image according to the fourth conversion matrix;

in the fourth index relationship table, looking up the table to obtain a first sagittal position Tx1 and a second sagittal position Tx2, and obtaining the storage positions of the sagittal image corresponding to Tx1 and Tx2, Tx1 and Tx2 being two sagittal planes closest to Tx, Tx being a sagittal position corresponding to ti, and ti being the target position in the coordinate system of the sagittal image;

selecting a sagittal plane closest to Tx from Tx1 and Tx2, displaying a measured sagittal image according to the corresponding storage position of the sagittal image, and obtaining a measured target position in the sagittal image in the measured sagittal image according to ti; and

constructing the planned position in the cross-sectional image to correspondingly obtain the planned position in the sagittal image.

Preferably, the method used for positioning needle correction is a spherical fitting method.

Preferably, the execution mechanism is a motion motor and or an energy generation apparatus.

Preferably, the execution mechanism is a motion motor and or an energy generation apparatus of a laser knife, an ultrasound knife, a water jet cutter and or an electrotome.

Another aspect of the present application is a biplanar ultrasound image planning apparatus, which is an apparatus for planning a biplanar ultrasound image photographed by an ultrasonic probe, uses the method according to any one of claims 1 to 15, and includes: an ultrasonic imaging module, a control module and a display module, where

the ultrasonic imaging module is configured to acquire a position of a marker and generate a cross-sectional image and a sagittal image;

the control module is configured to:

establish a coordinate system of the marker according to the position of the marker, and respectively and correspondingly establish a coordinate system of the cross-sectional image and a coordinate system of the sagittal image according to the positions of the cross-sectional image and the sagittal image,

calculate a first conversion matrix converted from the coordinate system of the cross-sectional image to the coordinate system of the marker, and a second conversion matrix converted from the coordinate system of the sagittal image to the coordinate system of the marker, and

calculate a third conversion matrix and or a fourth conversion matrix based on the first conversion matrix and the second conversion matrix; and

the display module is configured to: through the third conversion matrix and or the fourth conversion matrix, display a target position in the other coordinate system in a follow-up manner according to the target position in either of the coordinate system of the cross-sectional image and the coordinate system of the sagittal image.

At least one technical solution used by the embodiments of the present application can achieve the following beneficial effects:

1. According to the present application, by establishing a relationship between the ultrasonic biplanar image and the physical position of the ultrasonic probe, the accuracy and stability of diagnosing the biplanar ultrasound image or guiding surgical planning can be improved, and the diagnosis and surgery effect and safety can be enhanced. 2. According to the method of the present application, arbitrary interval planning can be achieved according to the changing rule of organic shape and the requirement of surgical planning due to the ability of the current computing and image acquisition devices. Compared with the convex array and the linear array completely independently acquiring images, an operator performs two-dimensional surgical planning based on a single-direction image or associates the positions of two linear array images based on the knowledge of human anatomy to assist in performing inaccurate three-dimensional surgical planning. The image follow-up planning provided by the present application can greatly improve the planning precision. 3. Through image follow-up acquisition and display during real-time planning, the manual operation process of the doctor is simplified and the efficiency is improved.

**BRIEF DESCRIPTION OF THE DRAWINGS**

The accompanying drawings described herein are used to provide a further understanding of the present application, and form part of the present application. Exemplary embodiments of the present application and descriptions thereof are used to explain the present application, and do not constitute any inappropriate limitation to the present application. In the drawings:

FIG. 1(a) is a method flowchart of a method embodiment of the present application;

FIG. 1(b) is a schematic diagram of a coordinate system of a probe of a method embodiment of the present application;

FIG. 1(c) is a schematic diagram of a spatial correction principle of a method embodiment of the present application;

FIG. 1(d) is a schematic diagram of a positioning needle correction principle of a method embodiment of the present application;

FIG. 1(e) is a schematic diagram of a coordinate system of a positioning needle of a method embodiment of the present application;

FIG. 2 is a flowchart of a method embodiment of the present application including image follow-up planning;

FIG. 3 is another flowchart of a method embodiment of the present application including image follow-up planning;

FIG. 4(a) is a schematic diagram of target position acquisition of a hyperplane ultrasound image follow-up planning embodiment;

FIG. 4(b) is a schematic diagram of a biplanar image coordinate of a hyperplane ultrasound image follow-up planning embodiment;

FIG. 5(a) is a method flow of a method embodiment of the present application including an execution flow;

FIG. 5(b) is a schematic diagram of a water jet cutter excision locus of a method embodiment of the present application including an execution flow;

FIG. 6 is a method flowchart of a method embodiment of the present application including a water jet cutter;

FIG. 7(a) is a schematic structural diagram of an apparatus embodiment of the present application;

FIG. 7(b) is another schematic structural diagram of an apparatus embodiment of the present application; and

FIG. 8 is another embodiment of an apparatus of the present application.

**DETAILED DESCRIPTION OF THE EMBODIMENTS**

To make the objectives, technical solutions and advantages of the present application clearer, the following clearly and completely describes the technical solutions of the present application with reference to the specific embodiments of the present application and the corresponding accompanying drawings Apparently, the described embodiments are merely some rather than all of the embodiments of the present application. All other embodiments obtained by a person of ordinary skill in the art based on the embodiments of the present application without creative efforts shall fall within the protection scope of the present application.

A doctor needs to repeatedly move the ultrasonic probe to observe the target position when manually using the biplanar ultrasound. The spatial position relationship between two planes is determined generally by identifying a structural key area in the image, which is time-consuming, laborious and poor in accuracy and stability.

Taking the prior art of water jet cutter resection for the hyperplastic prostate tissue as an example, an existing automatic water jet cutter planning method is rough in adjusting the corresponding movement of the boundary shape change of human organs, and a specific implementable method is not provided to establish an accurate corresponding relationship between the sagittal and cross-sectional images of the biplanar ultrasonic probe. Actually, an operator needs to manually acquire limited ultrasound image information. The whole organic is divided into several limited sections (such as three to four sections) based on the knowledge of human anatomy for parameter setting. The boundary change of the organ in the middle of each section only can be fitted through interpolation simulation. Furthermore, a specific method is not provided to reduce the fitting interval, thereby having an obvious error with the real organ boundary change curve.

The present application has the following innovation points. 1. The present application provides a method for determining the conversion between a biplanar ultrasound image and the physical position of an ultrasonic probe, which can acquire a relationship between two planar ultrasound images and the target position and is accurate in positioning and convenient to use. 2. The present application provides a method for planning follow-up acquisition and follow-up display of a biplanar ultrasound image. An ultrasonic probe adapter drives a dual-array ultrasonic probe to move automatically, which can be used to scan the whole organ and acquire continuous images of each cross section of the organ to accurately correspond to each position of the sagittal plane to achieve fine surgical planning, thereby achieving high-precision tissue ablation.

The technical solution provided by each embodiment of the present application will be described in detail below with reference to the accompanying drawings.

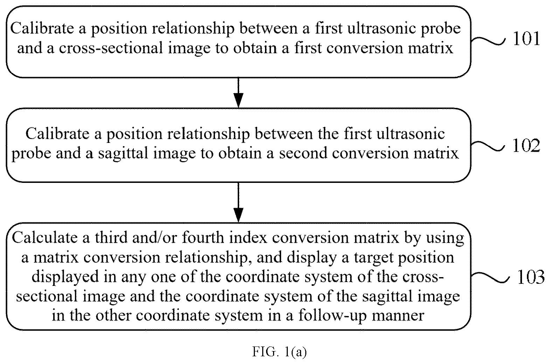

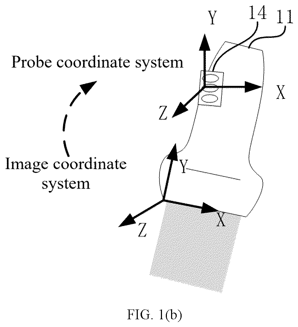

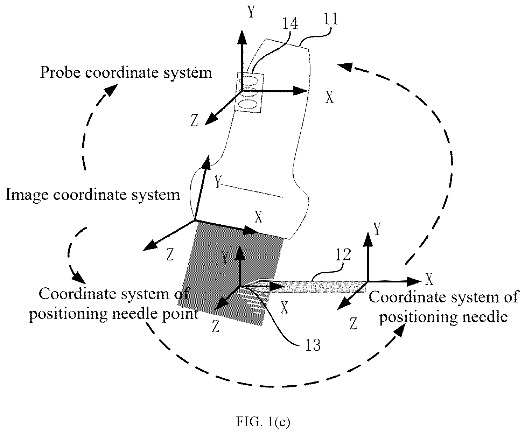

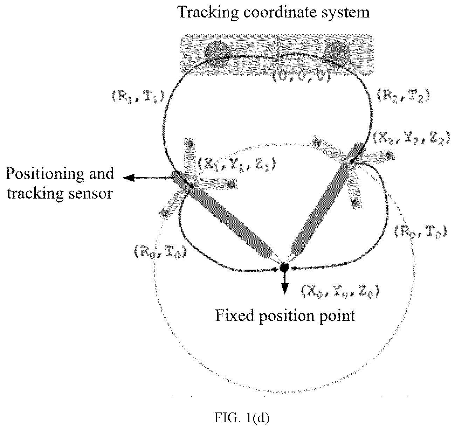

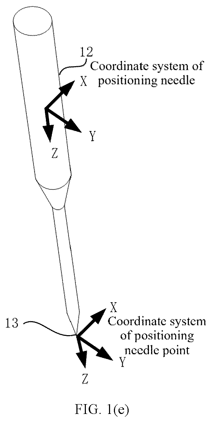

FIG. 1(a) is a method flowchart of a method embodiment of the present application. FIG. 1(b) is a schematic diagram of a coordinate system of a probe of a method embodiment of the present application. FIG. 1(c) is a schematic diagram of a spatial correction principle of a method embodiment of the present application. FIG. 1(d) is a schematic diagram of a positioning needle correction principle of a method embodiment of the present application. FIG. 1(e) is a schematic diagram of a coordinate system of a positioning needle of a method embodiment of the present application.

FIG. 1(b), FIG. 1(c) and FIG. 1(e) provide the description of each coordinate system of the embodiments of the present application.

As shown in FIG. 1(b), coordinate systems of an ultrasonic probe and an ultrasound image are provided. In FIG. 1(b), the ultrasonic probe 11 may be a biplanar ultrasonic probe, or may be a convex array probe or linear array probe.

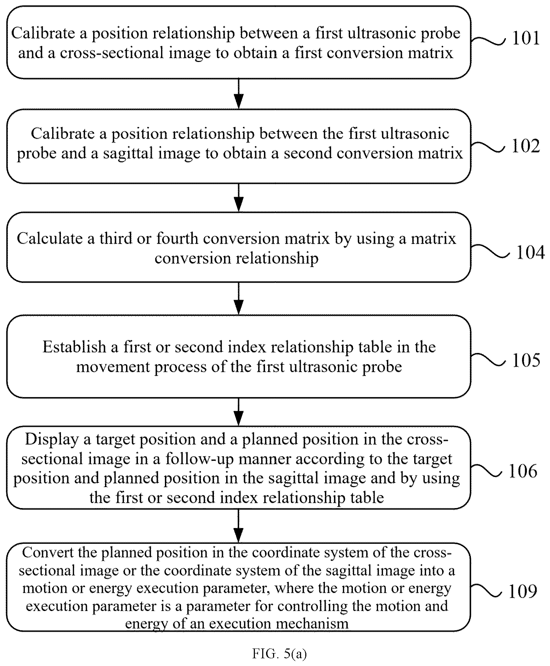

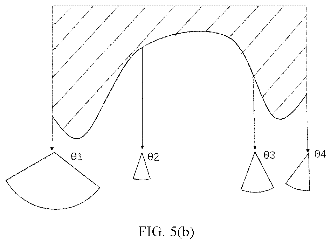

As shown in FIG. 1(b) and FIG. 1(c), a positioning and tracking sensor 14 is arranged in the ultrasonic probe 11, and a coordinate system established with the centroid of the positioning and tracking sensor as the origin is the coordinate system of the ultrasonic probe. It should be noted that if the ultrasonic probe in FIG. 1(b) and FIG. 1(c) is the biplanar ultrasonic probe, the coordinate system of the ultrasonic probe is the coordinate system of the biplanar ultrasonic probe, that is, a coordinate system of a marker in the embodiments of the present application. If the ultrasonic probe in FIG. 1(b) and FIG. 1(c) is the convex array probe or linear array probe, the coordinate system of the ultrasonic probe is the coordinate system of the convex array probe or linear array probe, corresponding to the coordinate system of the marker or the coordinate system of the second ultrasonic probe in the embodiment in FIG. 5 of the present application. Furthermore, the marker in the present application is not limited to the biplanar ultrasonic probe, the convex array probe and the linear array probe, and may further be a marked object imaged in the probe. The marked probe includes a plurality of marking points that can be imaged in the ultrasonic probe and have the known relative spatial position relationship on the marked object. That is, the marker in the present application is not limited to the biplanar ultrasonic probe, the convex array probe and the linear array probe, and may further be other objects containing features.

As shown in FIG. 1(b) and FIG. 1(c), an image coordinate system may be established at the position where the ultrasonic probe is connected to the human tissue. The coordinate system of the cross-sectional image is established in the Step 101, and the coordinate system of the sagittal image is established in the Step 102.

As shown in FIG. 1(c) and FIG. 1(e), a positioning needle point 13 is a needle point of a positioning needle 12, a needle body part of the positioning needle is provided with a positioning and tracking sensor, a coordinate system established with the positioning needle point as the origin is a coordinate system of the positioning needle point, and a coordinate system established with the centroid of the positioning and tracking sensor in the needle body of the positioning needle as the origin is a coordinate system of the positioning needle.

As shown in FIG. 1(b) and FIG. 1(c), each coordinate system has a conversion relationship. The image coordinate system (the coordinate system of the cross-sectional image or the coordinate system of the sagittal image) can be converted into the coordinate system of the ultrasonic probe, the image coordinate system can be converted into the coordinate system of the positioning needle point, the coordinate system of the positioning needle point can be converted into the coordinate system of the positioning needle, and the coordinate system of the positioning needle can be converted into the coordinate system of the ultrasonic probe.

It should be noted that each coordinate system in the present application may be an XYZ rectangular coordinate system, or may be other coordinate systems, without special explanation herein.

The method provided by the embodiment in FIG. 1(a) can be used for a biplanar ultrasonic probe. As the embodiment of the present application, a biplanar ultrasound image planning method specifically includes the following Steps 101-103.

Step 101: a position relationship between a marker and a cross-sectional image is calibrated to obtain a first conversion matrix, where the first conversion matrix is a matrix converted from a coordinate system of the cross-sectional image to a coordinate system of the marker.

It should be noted that in the embodiments of the present application, the marker in the Steps 101-103 is the biplanar ultrasonic probe and can generate the cross-sectional image and a sagittal image. A coordinate system established with the centroid of a positioning and tracking sensor as the origin is the coordinate system of the marker, also referred to as the coordinate system of the biplanar ultrasonic probe.

In the Step 101, the cross-sectional image is calibrated by the positioning and tracking sensor in the biplanar ultrasonic probe to determine the first conversion matrix $T_{Aim2p}$.

In the Step 101, the cross-sectional image may be calibrated by a spatial correction algorithm, which specifically includes the following Steps 101A-101E.

Step 101A: a fifth conversion matrix $T_{As2st}$ converted from a coordinate system of a positioning needle to a coordinate system of a positioning needle point is established through positioning needle correction.

In the Step 101A, positioning needle correction may be performed by a spherical fitting method, which specifically includes the following Steps 101AA-101AD.

As shown in FIG. 1(d), a positioning needle correction principle is provided, the positioning needle point can rotate around a fixed position point, and the positioning and tracking sensor is mounted on the positioning needle.

Step 101AA: the positioning needle point is fixed at the fixed position point, and the positioning needle is rotated slowly, so that the positioning and tracking sensor forms a spherical surface along with the rotation process, the center of the sphere is the position of the positioning needle point, and the radius is the distance from the positioning needle point to the centroid of the positioning and tracking sensor in the positioning needle.

As shown in FIG. 1(d), the coordinates of the fixed position point in a world coordinate system are $(X_0, Y_0, Z_0)$, the coordinates of the start position during rotation of the positioning needle are $(X_1, Y_1, Z_1)$, the coordinates of the end position during rotation of the positioning needle are $(X_2, Y_2, Z_2)$, and in the rotation process, the center of sphere is $T_0$ and the radius is $R_0$. (0, 0, 0) is the origin of the world coordinate system and is determined by a camera of the positioning needle. $(R_1, T_1)$ and $(R_2, T_2)$ respectively refer to R-axis and T-axis conversion matrices between a positioning apparatus of the positioning needle at any two different times and the world coordinate system in the rotation process of the positioning needle. It should be noted that the world coordinate system may be a local Cartesian coordinates coordinate system or other inertial coordinate systems.

Further, the position of the coordinate system of the positioning needle point is:

$$PP\_R = T_{M2R} \times PP\_M \qquad (1)$$

where PP_R is the coordinates of the positioning needle point in the world coordinate system, the world coordinate system is a fixed coordinate system, and the origin of the world coordinate system may be defined as the position where the positioning needle is first used; $T_{M2R}$ is a conversion matrix from the coordinate system formed by the centroid of the positioning and tracking sensor in the positioning needle to the world coordinate system, and the coordinate system formed by the centroid of the positioning and tracking sensor in the positioning needle is the coordinate system of the positioning needle; and PP_M is the coordinates of the positioning needle point in the coordinate system of the positioning needle.

Step 101AB: coordinate transformation is performed on the position of the coordinate system of the positioning needle point.

In the Step 101AB, the matrix $T_{M2R}$ may be decomposed into a rotation matrix and a displacement matrix. Therefore, the following coordinate transformation may be performed:

$$PP\_R = TR_{M2R} \times PP\_M + TV_{M2R} \qquad (2)$$

$$TR_{M2R} \times PP\_M - PP\_R = -TV_{M2R} \qquad (3)$$

$$TR_{M2R} \times I_{3 \times 3} \times PP\_M = PP\_R - TV_{M2R} \qquad (4)$$

where the formulas 2-4 are equivalent formulas, $TR_{M2R}$ is the rotation matrix of $T_{M2R}$, $TV_{M2R}$ is the displacement matrix of $TR_{M2R}$, and $I_{3 \times 3}$ is a 3*3 order unit matrix.

Step 101AC: PP_M and PP_R are solved by the least square method.

In the Step 101AC, the formula 4 is written as follows:

$$Ai \times xi = bi \qquad (5)$$

where Ai is a least square coefficient and Ai = $[TR_{M2R} | -I_{3 \times 3}]$, bi is a least square target value and bi = $-TV_{M2R}$, and xi is an unknown number of the least square target value and xi = [PP_M, PP_R].

The optimal solution of xi is solved by the least square method to obtain PP_M and PP_R.

Step 101AD: a conversion matrix $T_{s2st}$ converted from the coordinate system of the positioning needle to the coordinate system of the positioning needle point is calculated.

In the Step 101AD, assuming that the direction of the coordinate system of the positioning needle point is the same as the direction of the coordinate system of the positioning needle, a conversion matrix $T_{s2st}$ from the coordinate system of the positioning needle point to the coordinate system of the positioning needle may be obtained:

$$T_{s2st} = \begin{bmatrix} 1 & 0 & 0 & A_x \\ 0 & 1 & 0 & A_y \\ 0 & 0 & 1 & A_z \\ 0 & 0 & 0 & 1 \end{bmatrix} \qquad (6)$$

Ax, Ay and Az are respectively vectors of PP_M in x, y and z directions.

Step 101B: a sixth conversion matrix $T_{Ast2p}$ converted from the coordinate system of the positioning needle to the coordinate system of the biplanar ultrasonic probe is obtained through a positioning and tracking system.

In the Step 101B, the positioning and tracking system is configured to obtain the sixth conversion matrix through the positioning and tracking sensor on the positioning needle and the positioning and tracking sensor on the biplanar ultrasonic probe.

In the Step 101B, the positioning and tracking system may be a multi-target optical camera, or may be systems for positioning and tracking the positions of the positioning needle and the biplanar ultrasonic probe.

Step 101C: a seventh conversion matrix $T_{Ast2p}$ converted from the coordinate system of the positioning needle point to the coordinate system of the biplanar ultrasonic probe through the fifth conversion matrix and the sixth conversion matrix.

In the Step 101C, according to the matrix relationship $T_{As2p} = T_{As2st} \times T_{Ast2p}$, the seventh conversion matrix is calculated as:

$$T_{Ast2p} = T_{As2p} \times T_{As2st}^{-1} \qquad (7)$$

where $T_{As2st}^{-1}$ is an inverse matrix of $T_{As2st}$.

Step 101D: an eighth conversion matrix $T_{Ast2im}$ converted from the coordinate system of the positioning needle point to the coordinate system of the cross-sectional image is calculated according to a position relationship of the positioning needle in the cross-sectional image.

Step 101E: the first conversion matrix $T_{Aim2p}$ is calculated according to the seventh conversion matrix and the eighth conversion matrix.

In the Step 101E, according to the matrix relationship $T_{Ast2im} \times T_{Aim2p} = T_{Ast2p}$, the first conversion matrix is calculated as:

$$T_{Aim2p} = T_{Ast2im}^{-1} \times T_{Ast2p} \qquad (8)$$

where $T_{Ast2im}^{-1}$ is an inverse matrix of $T_{Ast2im}$.

Step 102: a position relationship between the biplanar ultrasonic probe and the sagittal image is calibrated to obtain a second conversion matrix, where the second conversion matrix is a matrix converted from the coordinate system of the sagittal image to the coordinate system of the biplanar ultrasonic probe.

In the Step 102, the position relationship between the biplanar ultrasonic probe and the sagittal image can be calibrated by a spatial correction method to obtain the second conversion matrix $T_{Bim2p}$.

It should be noted that the method for calculating the second conversion matrix is the same as the method for calculating the first conversion matrix in the Step 101, specifically including the following Steps 102A-102E.

Step 102A: a ninth conversion matrix $T_{Bs2st}$ converted from the coordinate system of the positioning needle to the coordinate system of the positioning needle point is established through positioning needle correction.

In the Step 102A, the ninth conversion matrix and the fifth conversion matrix may be the same matrix or different matrices, depending on the placing position and the placing angle of the positioning needle point.

Step 102B: a tenth conversion matrix $T_{Bst2p}$ converted from the coordinate system of the positioning needle to the coordinate system of the biplanar ultrasonic probe is obtained through the positioning and tracking system.

Step 102C: an eleventh conversion matrix $T_{Bst2p}$ converted from the coordinate system of the positioning needle point to the coordinate system of the biplanar ultrasonic probe is obtained through the ninth conversion matrix and the tenth conversion matrix:

$$T_{Bst2p} = T_{Bs2p} \times T_{Bs2st}^{-1} \qquad (9)$$

Step 102D: a twelfth conversion matrix $T_{Bst2im}$ converted from the coordinate system of the positioning needle point to the coordinate system of the sagittal image is calculated according to a position relationship of the positioning needle in the sagittal image.

Step 102E: the second conversion matrix $T_{Bim2p}$ is calculated according to the eleventh conversion matrix and the twelfth conversion matrix.

$$T_{Bim2p} = T_{Bst2im}^{-1} \times T_{Bst2p} \qquad (10)$$

Step 103: a third conversion matrix and or a fourth conversion matrix are/is calculated by a matrix conversion relationship, and a target position displayed in any one of the coordinate system of the cross-sectional image and the coordinate system of the sagittal image is displayed in the other coordinate system in a follow-up manner.

In the Step 103, the third conversion matrix is a matrix converted from the coordinate system of the cross-sectional image to the coordinate system of the sagittal image, and the fourth matrix is a matrix converted from the coordinate system of the sagittal image to the coordinate system of the cross-sectional image.

In the Step 103, the third conversion matrix is:

$$T_{Aim2Bim} = T_{Aim2p} \times T_{Bim2p}^{-1} \qquad (11),$$

and the fourth conversion matrix is:

$$T_{Bim2Aim} = T_{Bim2p} \times T_{Aim2p}^{-1} \qquad (12)$$

where $T_{Aim2Bim}$ is the third conversion matrix, $T_{Aim2Bim}$ is the fourth conversion matrix, $T_{Bim2p}^{-1}$ is the inverse matrix of $T_{Bim2p}$, and $T_{Aim2p}^{-1}$ is the inverse matrix of $T_{Aim2p}$.

In the Step 103, the target position may be displayed in the coordinate system of the cross-sectional image, and the target position of the target may be displayed in the coordinate system of the sagittal image in a follow-up manner, or the target position may be displayed in the coordinate system of the sagittal image, and the target position of the target may be displayed in the coordinate system of the cross-sectional image in a follow-up manner.

Further, the planned position displayed in any one of the coordinate system of the cross-sectional image and the coordinate system of the sagittal image is displayed in the other coordinate system in a follow-up manner.

It should be noted that the target position refers to the position of a target outline required to be displayed, the planned position refers to the outline position of a planned trajectory, and in practical use, boundary planning is performed according to the target position to obtain the planned position.

The embodiments of the present application provide a biplanar ultrasonic planning method. A position relationship conversion matrix of the ultrasonic biplanar probe and the image is established through image calibration, and the flow can be performed on a device once before clinical use without changing the hardware structure and the connection mode. The method can be used for a transrectal biplanar ultrasonic probe for prostate surgery navigation.

The embodiments of the present application can be used for image display scenarios. For example, a position relationship between two planar ultrasonic images and the actual tissue organ can be established in the surgery scenario using biplanar ultrasound, and images of two planes of the ultrasonic probe can be displayed accurately in a follow-up manner for diagnosing or cooperating with other surgical instruments in the images to arrive at the preoperative planned or intraoperative target position of the doctor.

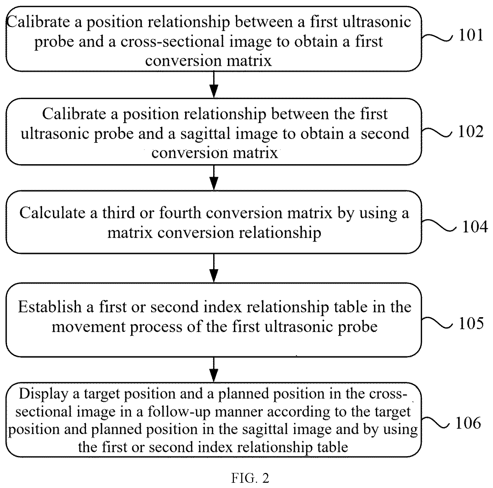

FIG. 2 is a flowchart of a method embodiment of the present application including image follow-up planning, which can be used for a surgical process to achieve the image follow-up planning of the cross-sectional image. As the embodiment of the present application, a biplanar ultrasonic image planning method specifically includes the following Steps 101-102 and 104-106.

In the real-time flow, a position relationship between the ultrasonic physical position and the image is established first by acquiring a biplanar ultrasonic image in real time, that is, an index relationship table in the flow. Then in the surgical planning, the cross-sectional image will be acquired or displayed in a follow-up manner according to the target position in the sagittal image and the target planning.

Step 101: a position relationship between the marker and the cross-sectional image is calibrated to obtain a first conversion matrix.

Step 102: a position relationship between the marker and the sagittal image is calibrated to obtain a second conversion matrix.

Step 104: a third conversion matrix and or a fourth conversion matrix are/is calculated by a matrix conversion matrix.

Step 105: a first index relationship table or a second index relationship table is established in the movement process of the ultrasonic probe, where the first index relationship table is an index relationship table of the physical position of the ultrasonic probe and the position of the cross section, and the second index relationship table is an index relationship table of storage position information of the cross-sectional image and the position of the cross section.

In the Step 105, the physical position of the ultrasonic probe refers to the actual physical position of the ultrasonic probe, the position of the cross section refers to a coordinate set of cross-section image positions acquired in real time, and the storage position information of the cross-sectional image refers to a prestored cross-sectional image.

In the Step 105, the ultrasonic probe moves from the physical position M1 of the ultrasonic probe to Mn under the control of an ultrasonic adapter, and in the movement process, the ultrasonic image of the cross section is acquired in real time according to a fixed step length, that is, the positions S1 to Sn of the cross section.

In the Step 105, a first index relationship table: [{S1: M1, ..., Sn: Mn}] is established, where the position of the cross section is in one-to-one correspondence with the physical position of the ultrasonic probe.

A second index relationship table: [{S1: ImgA1, ..., Sn: ImgAn}] is established, where ImgA1 to ImgAn represent the storage position information of the cross-sectional image, which is in one-to-one correspondence with the position of the cross section.

It should be noted that the position of the cross section and the position of the sagittal plane acquired at a certain moment represent images acquired by the biplanar ultrasonic probe at the same moment. The physical position of the cross-sectional image (or the position of the cross section) and the physical position of the sagittal image (or the position of the sagittal plane) are related to the mounting positions of the two probes, and the two positions are converted by the conversion matrix $T_{Aim2Bim}$ or $T_{Bim2Aim}$.

Step 106: the target position and planned position in the cross-sectional image are displayed in a follow-up manner according to the target position and planned position in the sagittal image and by using the first index relationship table or the second index relationship table.

In the Step 106, the cross-sectional image will be displayed in a follow-up manner according to the target position and planned position in the sagittal image, which is specifically implemented by one of the following two methods.

Method 1: the target position and planned position in the cross-sectional image can be displayed in a follow-up manner according to the target position and planned position in the sagittal image and by using the first index relationship table in the real-time surgical planning, which specifically includes the following Steps 106A-106D.

Step 106A: a pixel point position is calculated, a target position in the coordinate system of the sagittal image is acquired, and a target position in the coordinate system of the cross-sectional image is calculated according to the third conversion matrix.

In the Step 106A, the coordinates of the target position in the coordinate system of the sagittal image are ti, the coordinates of the target position in the coordinate system of the cross-sectional image are si, and $si = T_{Bim2Aim} \times ti$.

Step 106B: in the first index relationship table, the closest first cross-sectional position and second cross-sectional position are obtained by looking up the table, the coordinates Mx1 of the physical position of a first cross-sectional probe and the coordinates Mx2 of the physical position of a second cross-sectional probe are correspondingly obtained, and the coordinates Mx of the physical position of a target cross-sectional probe are obtained through interpolation, where the first and second cross sections are two cross sections closest to a cross section Sx corresponding to a target position si, and the cross-sectional positions corresponding to the two cross sections are respectively Sx1 and Sx2; and si is the target position in the coordinate system of the cross-sectional image, Sx is the corresponding cross-section position of si in the coordinate system of the cross-sectional image, and Sx1 and Sx2 are respectively the first and second cross-sectional positions.

It should be noted that the first cross-sectional position and the second cross-sectional position refer to a position coordinate set of cross-sectional images.

Step 106C: the ultrasonic probe is moved to the coordinates of the physical position of the target cross-sectional probe to obtain the cross-sectional image and the target position of the image.

In the Step 106C, the ultrasonic cross-sectional real-time image displayed at this time is the cross-sectional image corresponding to the target position of the sagittal plane. The coordinates of the target position in the cross-sectional image can be obtained according to the coordinates si of the target position.

Step 106D: the planned position in the sagittal image can be constructed in the real-time surgical planning to correspondingly obtain the planned position in the cross-sectional image.

In the Step 106D, after a planning curve is constructed on the sagittal plane in the real-time surgical planning, the system displays the cross-sectional image at all the planned position points on the sagittal plane in a follow-up manner and displays the position of the planned position on the cross-sectional image.

Method 2: the target position and planned position in the cross-sectional image can be displayed in a follow-up manner according to the target position and planned position in the sagittal image by using the second index relationship table in the real-time surgical planning, which specifically includes the following Steps 106E-106H.

Step 106E: a pixel point position is calculated, a target position in the coordinate system of the sagittal image is acquired, and a target position in the coordinate system of the cross-sectional image is calculated according to the third conversion matrix.

The Step 106E is the same as the Step 106A.

Step 106F: in the second index relationship table, the closest first cross-sectional position and second cross-sectional position are obtained by looking up the table, and the storage positions of the first and second cross-sectional images are correspondingly obtained, where the first and second cross sections are two cross sections closest to a cross section Sx corresponding to a target position si, and the cross-sectional positions corresponding to the two cross sections are respectively Sx1 and Sx2; and si is the target position in the coordinate system of the cross-sectional image, Sx is the corresponding cross-section position of si in the coordinate system of the cross-sectional image, and Sx1 and Sx2 are respectively the first and second cross-sectional positions.

Step 106G: a cross section closest to Sx is selected from the first and second cross sections, and the cross-sectional image and the target position in the image are displayed according to the storage position of the corresponding cross-sectional image.

In the Step 106G, the cross section is a cross section in the first and second cross sections closest to Sx, and the coordinates of the target position in the cross-sectional image can be obtained according to the coordinates si of the target position.

Step 106H: the planned position in the sagittal image is constructed in the real-time surgical planning to correspondingly obtain the planned position in the cross-sectional image.

The Step 106H is the same as the Step 106D.

In the embodiments of the present application, how to display the target position and the planned position in the cross-sectional image in a follow-up manner according target position and the planned position in the sagittal image is specifically described by taking the case where a transrectal biplane ultrasound image guides automatic prostate resection as an example.

For example, first, the ultrasonic probe is placed at the far point of a motion trajectory (relative to a device end), and the sagittal image range at this position can cover the required entire prostate tissue. This position is recorded as a motion zero point of an ultrasonic probe adapter. In the process that the cutting device automatically moves to perform tissue ablation, the ultrasonic probe should keep a fixed position at the zero point and provide a sagittal image for observation and monitoring in the surgical process.

Secondly, after the zero point position is determined, the ultrasonic probe adapter controls the ultrasonic probe to linearly move in a Z direction from the zero point position to a direction away from a human body, keeps the work of a convex array probe in the motion process and continuously acquires the cross-sectional image, and then returns to the zero point position after acquisition to enter a next planning step.

Then, the ultrasonic probe adapter has a position sensor, and the system records the Z-direction displacement of each cross-section image acquired by the sensor in the motion process.

Finally, surgical planning is performed on the sagittal image based on the zero point position probe, any point on the sagittal image is selected, and the cross-sectional image corresponding to the Z-direction coordinate position can be calculated by the above Step 105.

The ultrasonic adapter can be controlled according to the Step 106A to the Step 106D to move the ultrasonic probe to the target position to acquire and display a real-time ultrasonic cross-sectional image in a follow-up manner, or the acquired cross-sectional image can be displayed in a follow-up manner directly according to the position and according to the Step 106E to the Step 106H.

It should be noted that the error between the Z-coordinate position of the cross-sectional image and the Z-coordinate position selected by planning is determined by an image sampling rate in the motion and acquisition position of the cross-sectional image.

As an example of the natural cavity of the human body, the embodiment described in the present application takes the ultrasonic-guided prostate tissue ablation scenario for the rectal cavity as an example. However, those skilled in the art should understand that the system and control method of the present application can also be applied to other cavities (such as digestive tract, urinary tract, genital tract, nasal cavity, external auditory canal and canalis nasolacrimalis) and organs.

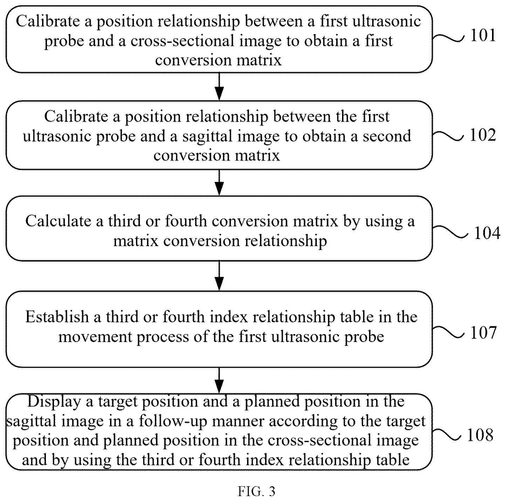

FIG. 3 is another flowchart of a method embodiment of the present application including image follow-up planning, which can be used for a surgical process to achieve the image follow-up planning of the sagittal image. As the embodiment of the present application, a biplanar ultrasonic image planning method specifically includes the following Steps 101, 102, 104 and 107-108.

Step 101: a position relationship between the marker and the cross-sectional image is calibrated to obtain a first conversion matrix.

Step 102: a position relationship between the marker and the sagittal image is calibrated to obtain a second conversion matrix.

Step 104: a third conversion matrix and or a fourth conversion matrix are/is calculated by a matrix conversion matrix.

Step 107: a third index relationship table or a fourth index relationship table is established in the movement process of the marker, where the third index relationship table is an index relationship table of the physical position of the marker and the position of the sagittal plane, and the fourth index relationship table is an index relationship table of the storage position information of the sagittal image and the position of the sagittal plane.

In the Step 107, the physical position of the ultrasonic probe refers to the actual physical position of the ultrasonic probe, the position of the sagittal plane refers to a coordinate set of sagittal image positions acquired in real time, and the storage position information of the sagittal image refers to a prestored sagittal image.

In the Step 107, the ultrasonic probe moves from the physical position M1 of the ultrasonic probe to Mn under the control of an ultrasonic adapter, and in the movement process, the ultrasonic image of the sagittal plane is acquired in real time according to a fixed step length, that is, the positions T1 to Tn of the sagittal plane.

In the Step 107, a third index relationship table: [{T1: M1, ..., Tn: Mn}] is established, where the position of the sagittal plane is in one-to-one correspondence with the physical position of the ultrasonic probe.

A fourth index relationship table: [{T1: ImgB1, ..., Tn: ImgBn}] is established, where ImgB1 to ImgBn represent the storage position information of the sagittal image, which is in one-to-one correspondence with the position of the sagittal plane.

It should be noted that the position of the cross section and the position of the sagittal plane acquired at a certain moment represent images acquired by the biplanar ultrasonic probe at the same moment. The physical position of the cross-sectional image (or the position of the cross section) and the physical position of the sagittal image (or the position of the sagittal plane) are related to the mounting positions of the two probes, and the two positions are converted by the conversion matrix $T_{Aim2Bim}$ or $T_{Bim2Aim}$.

Step 108: the target position and planned position in the sagittal image are displayed in a follow-up manner according to the target position and planned position in the cross-sectional image and by using the third index relationship table or the fourth index relationship table.

In the Step 108, the sagittal image will be displayed in a follow-up manner according to the target position and planned position in the cross-sectional image, which is specifically implemented by one of the following two methods.

Method 1: the target position and planned position in the sagittal image can be displayed in a follow-up manner according to the target position and planned position in the cross-sectional image and by using the third index relationship table in the real-time surgical planning, which specifically includes the following Steps 108A-108D.

Step 108A: a pixel point position is calculated, a target position in the coordinate system of the cross-sectional image is acquired, and a target position in the coordinate system of the sagittal image is calculated according to the fourth conversion matrix.

In the Step 108A, the coordinates of the target position in the coordinate system of the cross-sectional image are si, the coordinates of the target position in the coordinate system of the sagittal image are ti, and ti = $T_{Aim2Bim} \times$ si.

Step 108B: in the third index relationship table, a first sagittal position and a second sagittal position are obtained by looking up the table, the coordinates of the physical positions of the first and second sagittal probes are obtained correspondingly, and the coordinates of the physical position of a target sagittal probe are obtained through interpolation, where the first and second sagittal planes are two sagittal planes closest to the sagittal plane Tx corresponding to the target position ti, and Tx1 and Tx2 are the positions of the two sagittal planes; and ti is the target position in the coordinate system of the sagittal image, Tx is the position of the sagittal plane corresponding to ti, and Tx1 and Tx2 are respectively the first and second sagittal positions.

Step 108C: a first ultrasonic probe is moved to the coordinates of the physical position of the target sagittal probe to obtain the sagittal image and the target position in the image.

In the Step 108C, the ultrasonic sagittal real-time image displayed at this time is the sagittal image corresponding to the target position of the cross section. The coordinates of the target position in the sagittal image can be obtained according to the coordinates ti of the target position.

Step 108D: the planned position can be constructed on the cross-sectional image in the real-time surgical planning to correspondingly obtain the planned position in the sagittal image.

In the Step 108D, after a planning curve is constructed on the cross section in the real-time surgical planning, the system displays the sagittal image at all the planned position points on the cross section in a follow-up manner and displays the position of the planned position on the sagittal image.

Method 2: the target position and planned position in the sagittal image can be displayed in a follow-up manner according to the target position and planned position in the cross-sectional image by using the fourth index relationship table in the real-time surgical planning, which specifically includes the following Steps 108E-108H.

Step 108E: a pixel point position is calculated, a target position in the coordinate system of the cross-sectional image is acquired, and a target position in the coordinate system of the sagittal image is calculated according to the fourth conversion matrix.

The Step 108E is the same as the Step 108A.

Step 108F: in the fourth index relationship table, looking up the table to obtain a first sagittal position Tx1 and a second sagittal position Tx2, and obtaining the storage positions of the sagittal image corresponding to Tx1 and Tx2, where Tx1 and Tx2 are two sagittal planes closest to Tx, Tx is the sagittal position corresponding to ti, and ti is the target position in the coordinate system of the sagittal image.

Step 108G: a sagittal plane closest to Tx is selected from the first and second sagittal planes, and the sagittal image and the target position in the image are displayed according to the storage position of the corresponding sagittal image.

In the Step 108G, the sagittal plane is a sagittal plane of the first and second sagittal planes closest to Tx. The coordinates of the target position in the sagittal image can be obtained according to the coordinates ti of the target position.

Step 108H: the planned position can be constructed on the cross-sectional image in the real-time surgical planning to correspondingly obtain the planned position in the sagittal image.

The Step 108H is the same as the Step 108D.

In the embodiments in FIG. 2 and FIG. 3, the index relationship table is generated according to the relationship between two ultrasonic plane images at continuous positions acquired in real time and the actual spatial position, and according to the target position during the planning of one ultrasonic planar image, the other ultrasonic planar image at the corresponding position can be displayed in a follow-up manner.

In the embodiments in FIG. 2 and FIG. 3, the biplanar ultrasonic probe is fixed and controlled by the ultrasonic probe adapter with high-precision position feedback, so that accurate surgical planning can be achieved.

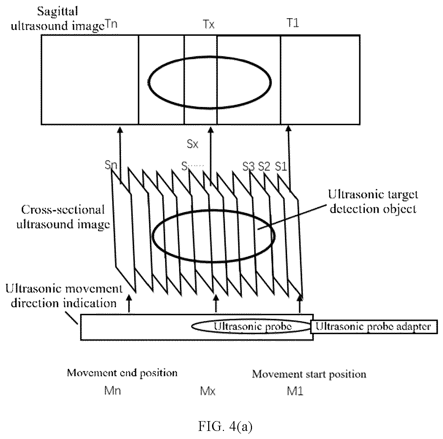

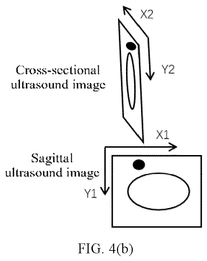

FIG. 4(a) is a schematic diagram of target position acquisition of a hyperplane ultrasound image follow-up planning embodiment; and FIG. 4(b) is a schematic diagram of a biplanar image coordinate of a hyperplane ultrasound image follow-up planning embodiment.

The physical positions of the first to fourth index relationships are shown in FIG. 4(a). As shown in FIG. 4(a), the ultrasonic probe moves from the coordinates M1 of the physical positions to Mn under the control of the ultrasonic adapter. It should be noted that the ultrasonic probe herein may be a convex array probe or linear array probe or biplanar ultrasonic probe.

In the embodiments of the present application, the ultrasonic probe is a biplanar ultrasonic probe, and the biplanar ultrasonic probe moves from the coordinates M1 of the physical position to Mn, where M1 is the start physical position of the biplanar ultrasonic probe, and Mn is the end physical position of the biplanar ultrasonic probe. In the movement process, the biplanar ultrasonic image is acquired in real time according to a fixed step length, that is, the positions S1 to Sn of the cross-sectional images in the figure represent a position coordinate set of each cross-sectional image, and the positions T1 to Tn of the sagittal images represent a position coordinate set of each sagittal image.

As shown in FIG. 4(a), the biplanar ultrasonic probe may generate a sagittal ultrasonic image shown in FIG. 4(a) at the position Mn. In the image, the elliptic outline represents an ultrasonic target detection object, and the position of the ultrasonic target detection object is the target position.

When the biplanar ultrasonic probe moves between M1 and Mn, the corresponding cross-sectional ultrasonic image may be generated, such as the positions S1-Sn of the cross-sectional real-time images in the figure.

In the movement process, the biplanar ultrasonic image is acquired in real time according to the fixed step length, that is, the cross-sectional real-time images S1 to Sn and the sagittal images T1 to Tn in the figure.

The following index tables are established:
a first index relationship table: {S1: M1, ..., Sn: Mn}], a second index relationship table: [{S1: ImgA1, ..., Sn: ImgAn}], a third index relationship table: [{T1: M1, ..., Tn: Mn}], and a fourth index relationship table: [{T1: ImgB1, ..., Tn: ImgBn}].

S1 is a start cross section, Sn is an end cross section, ImgA1 is a storage position of a start cross-sectional image, and ImgAn is a storage position of an end cross-sectional image. Correspondingly, T1 is a start sagittal surface, Tn is an end sagittal surface, ImgB1 is a storage position of a starting sagittal image, and ImgBn is a storage position of an ending sagittal image.

FIG. 4(b) shows a relative position relationship between the cross-sectional ultrasonic image and the sagittal ultrasonic image. The cross-sectional ultrasonic image and the sagittal ultrasonic image are orthogonal to each other, which can display two sections of a target (black spot in the figure).

The embodiments of the present application describe that the target position of the target can be displayed in the coordinate system of the cross-sectional image in a follow-up manner after the target position is displayed in the coordinate system of the sagittal image.

Further, the target position can also be displayed in the coordinate system of the cross-sectional image, and the target position can be displayed in the coordinate system of the sagittal image in a follow-up manner.

For example, the biplanar ultrasonic probe moves from the coordinates M1 of the physical position to Mn to obtain S1 to Sn of one cross-sectional image and T1 to Tn of one sagittal image. The target position is displayed on the cross-sectional image first, and the target position of the target is obtained on the sagittal image in a follow-up manner through the third index relationship table or the fourth index relationship table.

In the embodiments of the present application, the ultrasonic probe may be a biplanar ultrasonic probe, or the ultrasonic probe may be a discrete convex array probe and linear array probe.

If the ultrasonic probe is the linear array probe and convex array probe, corresponding index relationship tables are present: the first index relationship table may be expressed as: {S1: MA1, ..., Sn: MAn}], a second index relationship table: [{S1: ImgA1, ..., Sn: ImgAn}], a third index relationship table: [{T1: MB1, ..., Tn: MBn}], and a fourth index relationship table: [{T1: ImgB1, ..., Tn: ImgBn}]. MA1 is the starting physical position of the convex array probe, MAn is the ending physical position of the convex array probe, MB1 is the start physical position of the linear array probe, and MBn is the end physical position of the linear array probe.

FIG. 5(a) is a method flow of a method embodiment of the present application including an execution flow; and FIG. 5(b) is a schematic diagram of a water jet cutter excision locus of a method embodiment of the present application including an execution flow, which can be applied to the workflow of the water jet cutter.

In the embodiments of the present application, a biplanar ultrasonic image planning method specifically includes the following Steps 101-102, 104-106 and 109.

Step 101: a position relationship between a marker and a cross-sectional image is calibrated to obtain a first conversion matrix, where the first conversion matrix is a matrix converted from a coordinate system of the cross-sectional image to a coordinate system of the marker.

Step 102: a position relationship between the marker and the sagittal image is calibrated to obtain a second conversion matrix.

Step 104: a third conversion matrix and or a fourth conversion matrix are/is calculated by a matrix conversion matrix.

Step 105: a first index relationship table or a second index relationship table is established in the movement process of the ultrasonic probe, where the first index relationship table is an index relationship table of the physical position of the ultrasonic probe and the position of the cross section, and the second index relationship table is an index relationship table of storage position information of the cross-sectional image and the position of the cross section.

Step 106: the target position displayed in any one of the coordinate system of the cross-sectional image and the coordinate system of the sagittal image is displayed in the other coordinate system in a follow-up manner.

Step 109: the planned position in the coordinate system of the cross-sectional image or the coordinate system of the sagittal image is converted into a motion and or energy execution parameter.

It should be noted that the motion execution parameter is a parameter for controlling an execution mechanism to move, and the energy execution parameter is a parameter for controlling the execution mechanism to release energy, for example, water jet cutting is a process of releasing energy, and water jet is a process of releasing energy.

In the Step 109, after the planned position is converted into the motion execution parameter and or the energy execution parameter, the execution mechanism may be manually controlled to move according to the planned position and execute according to the planned energy, or may be automatically controlled to move according to the planned position and execute according to the planned energy.

It should be noted that the planned energy refers to planned energy for the execution mechanism to generate and release.

An execution module may be a water jet cutter, an electrotome, a laser knife, an ultrasound knife or other execution mechanisms, which is not particularly limited here.

For example, after the planned position is converted into the motion and energy execution parameters, the water jet cutter or other execution mechanisms are manually controlled to perform movement and resection along the planned position. For another example, after the planned position is converted into the motion execution parameter, the water jet cutter or other execution mechanisms are automatically controlled to perform movement and resection along the planned position through a motor and an energy generation apparatus of the execution mechanism.

In the Step 109, the water jet cutting trajectory on the generated biplanar ultrasonic image is shown in FIG. 5(b), where the shaded part is the water jet cutting trajectory on the sagittal image, $\theta1$ to $\theta4$ are cross-sectional ultrasonic fan-shaped trajectories at the corresponding positions on the sagittal plane, and the fan-shaped position can be linked and planned at any position on the sagittal plane. The generated trajectory description is shown as follows:

S_img = [([y0_start, z0_start], [y0_end, z0_end], $\theta$0_start, $\theta$0_end), ([y1_start, z1_start], [y1_end, z1_end], $\theta$1_start, $\theta$1_end), (13), ..., and ([yn_start, zn_start], [yn_end,zn_end], $\theta$n_start, $\theta$n_end)]

S_img represents the water jet cutting trajectory on the biplanar ultrasonic image, and ([y0_start, z0 start], [y0_end, z0_end], [$\theta$0_start, $\theta$0_end]) represents the range of the sagittal plane and the cross section in a step length movement voxel required to control water jet cutting. y0_start and y0_end respectively represent a start value and an end value of the y-axis coordinates of the sagittal plane of water jet cutting in the $0^{th}$ movement voxel. z0_start and z0_end respectively represent a start value and an end value of the z-axis coordinates of the sagittal plane of water jet cutting in the $0^{th}$ movement voxel. $\theta$0_start and $\theta$0_end respectively represent a start value and an end value of a cross-sectional included angle of water jet cutting in the $0^{th}$ movement voxel.

It should be noted that xy plane represents the cross section, and yz plane represents the sagittal plane.

In the Step 109, a motion model of a water jet cutter mechanism is constructed according to a physical parameter of the water jet cutter mechanism to obtain a conversion relationship matrix Ts from the coordinates of the spatial position of the end movement of the water jet cutter to the actual control parameter of the motor, then the motion execution parameter S_motor is expressed as follows:

$$S\_motor = Ts \times S\_img \qquad (14)$$

S_motor represents the motion execution parameter, and the resection action can be completed by controlling the water jet cutter or other execution mechanisms to move according to the parameter trajectory S_motor. The energy of the water jet cutter can be controlled through the movement of the water jet cutter energy generation apparatus, and the conversion relationship has been included in Ts.

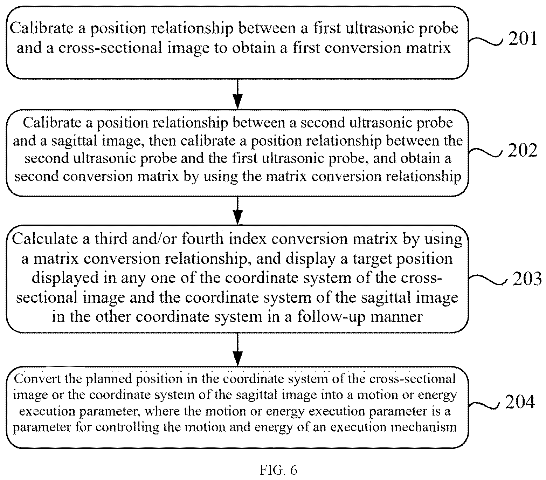

FIG. 6 is a method flowchart of a method embodiment of the present application including a water jet cutter.

It should be noted that in the embodiments of the present application, the marker is a convex array probe, and the coordinate system of the marker is the coordinate system of the convex array probe; and the second ultrasonic probe is a linear array probe, and the coordinate system of the second ultrasonic probe is the coordinate system of the linear array probe. The convex array probe and the linear array probe are two independent probes, which can perform coordinate system calibration during use.

In the embodiments of the present application, a biplanar ultrasonic image planning method specifically includes the following Steps 201-204.

Step 201: a position relationship between the marker and the cross-sectional image is calibrated to obtain a first conversion matrix.

In the Step 201, the first conversion matrix is a matrix converted from the coordinate system of the cross-sectional image to the coordinate system of the convex array probe.

The method for calculating the first conversion matrix in the Step 201 is the same as that in the Step 101, which is not elaborated herein.

Step 202: the position relationship between the second ultrasonic probe and the sagittal image is calibrated, the position relationship between the second ultrasonic probe and the marker is calibrated, and the second conversion matrix is obtained by the matrix conversion relationship.

In the Step 202, the position relationship between the linear array probe and the sagittal image is calibrated, the position relationship between the linear array probe and the convex array probe is calibrated, and the second conversion matrix can be obtained. In the embodiments of the present application, the second conversion matrix is a matrix converted from the coordinate system of the sagittal image to the coordinate system of the convex array probe.

The method for calibrating the position relationship between the linear array probe and the sagittal image in the Step 202 is the same as that in the Step 102, which is not elaborated herein.

In the Step 202, the method for calibrating the position relationship between the linear array probe and the convex array probe may be: performing calibration by converting the coordinate system of the linear array probe and the convex array probe into the same fixed coordinate system, or may be: directly converting the coordinate system of the linear probe into the coordinate system of the convex array probe or converting the coordinate system of the convex array probe into the coordinate system of the linear array probe.

It should be noted that the coordinate system of the convex array probe refers to a coordinate system established by taking the centroid of a positioning and tracking sensor in the convex array probe as the origin, and the coordinate system of the linear array probe refers to a coordinate system established by taking the centroid of a positioning and tracking sensor in the linear array probe as the origin.

Step 203: a third conversion matrix and or a fourth conversion matrix are/is calculated by a matrix conversion relationship, and a target position and a planned position displayed in any one of the coordinate system of the cross-sectional image and the coordinate system of the sagittal image are displayed in the other coordinate system in a follow-up manner.

In the Step 203, the target position and the planned position can be displayed in the coordinate system of the cross-sectional image, and the target position and the planned position can be displayed in the coordinate system of the sagittal image in a follow-up manner. Or the target position and the planned position can be displayed in the coordinate system of the sagittal image, and the corresponding target position and planned position can be displayed in the coordinate system of the cross-sectional image in a follow-up manner.

The method for planning a trajectory in one coordinate system and displaying the trajectory in the other coordinate system in a follow-up manner is the same as that in the Step 203, which is not elaborated herein.

Step 204: a planned position in the coordinate system of the cross-sectional image or the coordinate system of the sagittal image is converted into a motion or energy execution parameter, where the motion or energy execution parameter is a parameter for controlling the motion and energy of an execution mechanism.

The method for obtaining the motion or energy execution parameter in the Step 204 is the same as that in the Step 109, which is not elaborated herein.

In the Step 204, surgical planning is performed on the sagittal image based on the zero point position probe, any point on the sagittal image is selected, and the cross-sectional image corresponding to the Z-direction coordinate position can be calculated by the Steps 201-203.

The ultrasonic adapter can be controlled according to the Step 106 in the method 1 to move the ultrasonic probe to the target position to acquire and display a real-time ultrasonic cross-sectional image in a follow-up manner, or the acquired cross-sectional image can be displayed in a follow-up manner directly according to the position and according to the Step 106 in the method 2. The error between the Z-coordinate position of the cross-sectional image and the Z-coordinate position selected by planning is determined by an image sampling rate in the motion and acquisition position of the cross-sectional image.

According to the embodiments of the present application, the relative position of the rectal ultrasound biplane is accurately calibrated, a relative position relationship of images (cross section and sagittal plane) respectively generated by two probe arrays at the same motion position can be obtained through calculation, and a position conversion matrix can be generated.

The embodiments of the present application provide a water jet cutting process. The automatic water jet cutting process can be implemented according to the trajectory positions in the linear array probe and the convex array probe, so that the water jet cutting process is more accurate. It should be noted that the water jet cutting process of the present application can further be implemented through a biplanar ultrasonic probe or a three-dimensional probe.

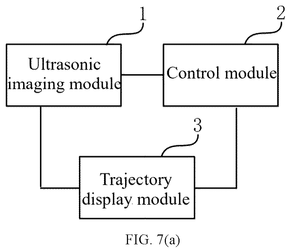

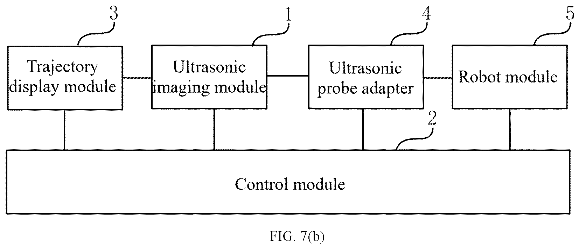

FIG. 7(a) is a schematic structural diagram of an apparatus embodiment of the present application; and FIG. 7(b) is another schematic structural diagram of an apparatus embodiment of the present application, which can be used to implement the method according to any embodiment of the present application.

A biplanar ultrasound image planning apparatus includes an ultrasonic imaging module 1, a control module 2 and a display module 3.

The ultrasonic imaging module is configured to acquire a position of a marker and generate a cross-sectional image and a sagittal image.

The control module is configured to: establish a coordinate system of the marker according to the position of the marker, and respectively and correspondingly establish a coordinate system of the cross-sectional image and a coordinate system of the sagittal image according to the positions of the cross-sectional image and the sagittal image; calculate a first conversion matrix converted from the coordinate system of the cross-sectional image to the coordinate system of the marker, and a second conversion matrix converted from the coordinate system of the sagittal image to the coordinate system of the marker; and calculate a third conversion matrix and or a fourth conversion matrix by a matrix conversion matrix.

The display module is configured to: according to a target position in any one of the coordinate system of the cross-sectional image and the coordinate system of the sagittal image, display a target position in the other coordinate system in a follow-up manner.

In the embodiments of the present application, the ultrasonic imaging module is an intracavity biplanar ultrasonic probe system, and the control system controls the action of each module to calculate, generate and store each conversion matrix and each index relationship table.

Further, in the embodiments of the present application, the ultrasonic imaging module includes: a biplanar ultrasonic probe and a positioning needle, where the marker is the biplanar ultrasonic probe.

A positioning and tracking sensor is arranged in the biplanar ultrasonic probe and configured to acquire the position of the biplanar ultrasonic probe. A positioning and tracking sensor is arranged in a needle body of the positioning needle and configured to acquire the position of the positioning needle and the position of a positioning needle point.

The control module is further configured to: establish a coordinate system of the positioning needle to a coordinate system of the positioning needle point according to the positions of the positioning needle and the positioning needle point; and calculate a fifth conversion matrix by a positioning needle correction method and calculate a sixth to eighth conversion matrices so as to obtain the first conversion matrix.

In the embodiments of the present application, as shown in FIG. 7(b), a biplanar ultrasonic image planning apparatus includes: an ultrasonic imaging module 1, a control module 2, a display module 3, an ultrasonic adapter probe 4 and a robot module 5.

The biplanar ultrasonic probe in the ultrasonic imaging module can be fixed through the ultrasonic probe adapter, and the degrees of freedom in at least two directions of straight line and rotation are provided for the biplanar ultrasonic probe. The biplanar ultrasonic probe and the ultrasonic probe adapter can be fixed through the robot module.

Further, the control module is further configured to: establish a first index relationship table or a second index relationship table in the movement process of the biplanar ultrasonic probe; and display the target position and planned position in the cross-sectional image in a follow-up manner according to the target position and planned position in the sagittal image and by using the first index relationship table or the second index relationship table in the real-time surgical planning.

Further, the control module is further configured to: establish a third index relationship table or a fourth index relationship table in the movement process of the ultrasonic probe; and display the target position and planned position in the sagittal image in a follow-up manner according to the target position and planned position in the cross-sectional image and by using the third index relationship table or the fourth index relationship table in the real-time surgical planning.

The specific methods for implementing the functions of the ultrasonic imaging module, the control module and the trajectory display module are as described in various method embodiments of the present application, which will not be elaborated herein.

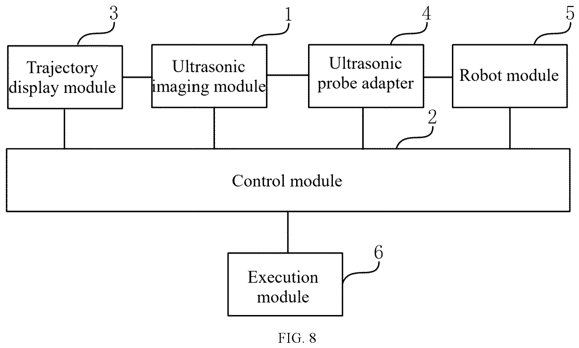

FIG. 8 is another embodiment of an apparatus of the present application, which can use the method according to any embodiment of the present application.

A biplanar ultrasound image planning apparatus includes: an ultrasonic imaging module 1, a control module 2, a trajectory display module 3, an ultrasonic adapter probe 4, a robot module 5 and an execution module 6.

The ultrasonic imaging module is configured to acquire positions of a convex array probe and a linear array probe and generate a cross-sectional image and a sagittal image.

The control module is configured to: respectively establish a coordinate system of the convex array probe and a coordinate system of the linear array probe according to the positions of the convex array probe and the linear array probe, and respectively establish a coordinate system of the cross-sectional image and a coordinate system of the sagittal image according to the positions of the cross-sectional image and the sagittal image.

The control module is further configured to calculate a first conversion matrix converted from the coordinate system of the cross-sectional image to the coordinate system of the convex array probe, and a second conversion matrix converted from the coordinate system of the sagittal image to the coordinate system of the linear array probe; and calculate a third conversion matrix and or a fourth conversion matrix by a matrix conversion matrix.

The ultrasonic probe adapter is configured to fix the convex array probe or the linear array probe in the ultrasonic imaging module, and provide the degrees of freedom in at least two directions of straight line and rotation for the convex array probe and the linear array probe. The robot module is configured to fix the convex array probe, the linear array probe and the ultrasonic probe adapter.

The execution module is configured to convert the planned position in the coordinate system of the cross-sectional image or the coordinate system of the sagittal image into motion and energy execution parameters, and control an execution mechanism to move and generate energy.

Preferably, the execution module may be a water jet cutter, an electrotome, a laser knife, an ultrasound knife or other execution mechanisms. The execution module may be controlled manually or automatically to move and perform resection. If the execution module is controlled automatically to move and perform resection, a motor of the execution module is driven to automatically control the execution module to move and automatically control the energy of an energy generation apparatus.

In some embodiments of the present application, the ultrasonic imaging module includes: a convex array probe and a linear array probe. The convex array probe is configured to acquire the position of the marker and the position of the cross-sectional image. The linear array probe is configured to acquire the position of the second ultrasonic probe and the position of the cross-sectional image.

The control module is further configured to establish a coordinate system of the second ultrasonic probe, calibrate the position relationship between the second ultrasonic probe and the sagittal image, calibrate the position relationship between the second ultrasonic probe and the marker, and perform calculation by the matrix conversion relationship to obtain the second conversion matrix.

According to the present application, the biplanar ultrasonic probe is fixed and controlled by the ultrasonic probe adapter with high-precision position feedback, so that accurate surgical planning can be achieved.

According to the embodiments of the present application, the ultrasonic probe adapter drives a dual-array ultrasonic probe to move automatically, scans the whole organ and acquires continuous images of each cross section of the organ to accurately correspond to each position of the sagittal plane to achieve fine surgical planning, thereby implementing high-precision tissue ablation, performing fine tissue ablation along the actual boundary of the organ, achieving an ideal surgical effect, and avoiding the surgical planning error of the organ model caused by manual acquisition of a limited ultrasonic image.

Those skilled in the art should understand that the embodiments of the present application may be provided as a method, a system, or a computer program product. Therefore, the present application may take the form of an entirely hardware embodiment, an entirely software embodiment, or an embodiment combining software and hardware aspects. In a typical configuration, a device of the present application includes one or more processors (one of CPU, FGAP and MUC), input/output user interfaces, network interfaces and memories.

Moreover, the present application may use a form of a computer program product that is implemented on one or more computer-usable storage media (including but not limited to a disk memory, a CD-ROM, an optical memory and the like) that include computer-usable program code.

Therefore, the present application further provides a computer-readable medium, where the computer-readable medium stores a computer program; and when the computer program is executed by a processor, the steps of the method according to any embodiment of the present application are implemented. For example, the memory of the present application may include a non-permanent memory, a random access memory (RAM), a non-volatile memory and or the like in the computer-readable medium, such as a read-only memory (ROM) or a flash RAM.

The computer-readable medium includes permanent and non-permanent, removable and non-removable media, and may store information by using any method or technology. The information may be computer-readable instructions, data structures, modules of programs or other data. Examples of the computer storage medium include but not limited to: a phase-change memory (PRAM), a static random access memory (SRAM), a dynamic random access memory (DRAM), another type of random access memory (RAM), a read-only memory (ROM), an electrically erasable programmable read-only memory (EEPROM), a flash memory or another memory technology, a compact disc read-only memory (CD-ROM), a digital video disc (DVD) or another optical memory, a magnetic cassette, a magnetic disk storage, another magnetic storage device, and any other non-transmission media that may be configured to store information that can be accessed by a computing device. As defined in this specification, the computer-readable medium does not include computer-readable transitory media, such as modulated data signals and carriers.

It should be noted that the terms "comprise", "include" and any other variants thereof are intended to cover non-exclusive inclusion, so that a process, a method, a commodity or a device that includes a series of elements not only includes these very elements, but may further include other elements not expressly listed, or further include elements inherent to this process, method, commodity or device. **In** the absence of more limitations, an element defined by "include a ..." does not exclude other same elements existing in the process, method or device including the element.

The above is only an embodiment of the present application and is not intended to limit the present application. For those skilled in the art, the present application may have various modifications and changes. Any modifications, equivalent substitutions, improvements and the like made within the spirit and principle of the present application should be included within the scope of the claims of the present application.

## Claims

1. A biplanar ultrasound image planning method, which is a biplanar ultrasound image planning method captured by an ultrasonic probe, **characterized in that**, and comprising the following steps:

   calibrating a position relationship between a marker and a cross-sectional image to obtain a first conversion matrix, the first conversion matrix being a matrix converted from a coordinate system of the cross-sectional image to a coordinate system of the marker;
   calibrating a position relationship between the marker and a sagittal image to obtain a second conversion matrix, the second conversion matrix being a matrix converted from a coordinate system of the sagittal image to a coordinate system of the marker;
   calculating a third conversion matrix and or a fourth conversion matrix based on the first conversion matrix and the second conversion matrix, the third conversion matrix being a matrix converted from the coordinate system of the cross-sectional image to the coordinate system of the sagittal image, and the fourth matrix being a matrix converted from the coordinate system of the sagittal image to the coordinate system of the cross-sectional image; and
   through the third conversion matrix and or the fourth conversion matrix, displaying a target position displayed in any of the coordinate system of the cross-sectional image and the coordinate system of the sagittal image in the other coordinate system in real time and in a follow-up manner.

2. The biplanar ultrasound image planning method according to claim 1, **characterized in that**, the marker is a biplanar ultrasonic probe.

3. The biplanar ultrasound image planning method according to claim 1, **characterized in that**, the marker is either a convex array probe for photographing the cross-sectional image or a linear array probe for photographing the sagittal image.

4. The biplanar ultrasound image planning method according to claim 1, **characterized in that**, the step of displaying in the other coordinate system further comprises:

   establishing a first index relationship table or a second index relationship table in the movement process of the ultrasonic probe, the first index relationship table being an index relationship table of a physical position of the ultrasonic probe and a cross-sectional position, and the second index relationship table being an index relationship table of storage position information of the cross-sectional image and the cross-sectional position; and
   displaying a target position and a planned position in the cross-sectional image in a follow-up manner by means of the first index relationship table or the second index relationship table and according to a target position and a planned position in the sagittal image.

5. The biplanar ultrasound image planning method according to claim 1, **characterized in that**, the step of displaying in the other coordinate system further comprises:

establishing a third index relationship table or a fourth index relationship table in the movement process of the ultrasonic probe, the third index relationship table being an index relationship table of a physical position of the ultrasonic probe and a sagittal position, and the fourth index relationship table being an index relationship table of storage position information of the sagittal image and the sagittal position; and

displaying a target position and a planned position in the sagittal image in a follow-up manner by means of the third index relationship table or the fourth index relationship table and according to a target position and a planned position in the cross-sectional image.

6. The biplanar ultrasound image planning method according to claim 2 or 3, **characterized in that**, calibrating the position relationship between the marker and the cross-sectional image by a spatial correction method to obtain the first conversion matrix specifically comprises:

establishing a fifth conversion matrix $T_{As2st}$ converted from a coordinate system of a positioning needle to a coordinate system of a positioning needle point through positioning needle correction;

obtaining a sixth conversion matrix $T_{As2p}$ converted from the coordinate system of the positioning needle to the coordinate system of the marker through a positioning and tracking system;

calculating a seventh conversion matrix $T_{Ast2p}$ converted from the coordinate system of the positioning needle point to the coordinate system of the marker through the fifth conversion matrix and the sixth conversion matrix;

calculating an eighth conversion matrix $T_{Ast2im}$ converted from the coordinate system of the positioning needle point to the coordinate system of the cross-sectional image according to a position relationship of the positioning needle in the cross-sectional image; and

calculating the first conversion matrix $T_{Aim2p}$ according to the seventh conversion matrix and the eighth conversion matrix.

7. The biplanar ultrasound image planning method according to claim 2 or 3, **characterized in that**,

calculating the position relationship between the marker and the sagittal image by a spatial correction method to obtain the second conversion matrix,

or performing inverse matrix operation on the first conversion matrix to obtain the second conversion matrix.

8. The biplanar ultrasound image planning method according to claim 1, **characterized in that**,

further comprising:

converting a planned position in the coordinate system of the cross-sectional image or the coordinate system of the sagittal image into a motion or energy execution parameter, the motion or energy execution parameter being a parameter for controlling the motion and energy of an execution mechanism.

9. The biplanar ultrasound image planning method according to claim 4, **characterized in that**,

the step of displaying the target position and the planned position in the cross-sectional image in a follow-up manner according to the target position and the planned position in the sagittal image further comprises:

calculating a pixel point position, acquiring a target position in the coordinate system of the sagittal image, and calculating a target position in the coordinate system of the cross-sectional image according to the third conversion matrix;

in the first index relationship table, looking up the table to obtain a first cross-sectional position Sx1 and a second cross-sectional position Sx2, obtaining the physical positions of the ultrasonic probe corresponding to Sx1 and Sx2, and obtaining a physical position of a target cross-sectional probe through interpolation, Sx1 and Sx2 being respectively positions of two cross sections closest to Sx, Sx being a cross-sectional position corresponding to si, and si being the target position in the coordinate system of the cross-sectional image;

moving the ultrasonic probe to the physical position of the target cross-sectional probe to obtain a measured cross-sectional image, and obtaining a measured target position in the cross-sectional image in the measured cross-sectional image according to si; and

constructing the planned position in the sagittal image to correspondingly obtain the planned position in the cross-sectional image.

10. The biplanar ultrasound image planning method according to claim 4, **characterized in that**,

the step of displaying the target position and the planned position in the cross-sectional image in a follow-up manner according to the target position and the planned position in the sagittal image further comprises:

calculating a pixel point position, acquiring a target position in the coordinate system of the sagittal image, and calculating a target position in the coordinate system of the cross-sectional image according to the third conversion matrix;

in the second index relationship table, looking up the table to obtain a first cross-sectional position Sx1 and a second cross-sectional position Sx2, and obtaining the storage positions of the cross-sectional image corresponding to Sx1 and Sx2, Sx1 and Sx2 being respectively positions of two cross sections closest to Sx, Sx being a cross-sectional position corresponding to si, and si being the target position in the coordinate system of the cross-sectional image;

selecting a cross section closest to Sx from Sx1 and Sx2, displaying a measured cross-sectional image according to the corresponding storage position of the cross-sectional image, and obtaining a measured target position in the cross-sectional image in the measured cross-sectional image according to si; and

constructing the planned position in the sagittal image to correspondingly obtain the planned position in the cross-sectional image.

**11.** The biplanar ultrasound image planning method according to claim 5, **characterized in that**, the step of displaying the target position and the planned position in the sagittal image in a follow-up manner according to the target position and the planned position in the cross-sectional image further comprises:

calculating a pixel point position, acquiring a target position in the coordinate system of the cross-sectional image, and calculating a target position in the coordinate system of the sagittal image according to the fourth conversion matrix;

in the third index relationship table, looking up the table to obtain a first sagittal position Tx1 and a second sagittal position Tx2, obtaining the physical positions of the ultrasonic probe corresponding to Tx1 and Tx2, and obtaining a physical position of a target sagittal probe through interpolation, Tx1 and Tx2 being positions of two sagittal planes closest to Tx, Tx being a sagittal position corresponding to ti, and ti being the target position in the coordinate system of the sagittal image;

moving the ultrasonic probe to the physical position of the target sagittal probe to obtain a measured sagittal image, and obtaining a measured target position in the sagittal image in the measured sagittal image according to ti; and

constructing the planned position in the cross-sectional image to correspondingly obtain the planned position in the sagittal image.

**12.** The biplanar ultrasound image planning method according to claim 5, **characterized in that**, the step of displaying the target position and the planned position in the sagittal image in a follow-up manner according to the target position and the planned position in the cross-sectional image further comprises:

calculating a pixel point position, acquiring a target position in the coordinate system of the cross-sectional image, and calculating a target position in the coordinate system of the sagittal image according to the fourth conversion matrix;

in the fourth index relationship table, looking up the table to obtain a first sagittal position Tx1 and a second sagittal position Tx2, and obtaining the storage positions of the sagittal image corresponding to Tx1 and Tx2, Tx1 and Tx2 being two sagittal planes closest to Tx, Tx being a sagittal position corresponding to ti, and ti being the target position in the coordinate system of the sagittal image;

selecting a sagittal plane closest to Tx from Tx1 and Tx2, displaying a measured sagittal image according to the corresponding storage position of the sagittal image, and obtaining a measured target position in the sagittal image in the measured sagittal image according to ti; and

constructing the planned position in the cross-sectional image to correspondingly obtain the planned position in the sagittal image.

**13.** The biplanar ultrasound image planning method according to claim 6, **characterized in that**, the method used for positioning needle correction is a spherical fitting method.

**14.** The biplanar ultrasound image planning method according to claim 8, **characterized in that**, the execution mechanism is a motion motor and or an energy generation apparatus.

15. The biplanar ultrasound image planning method according to claim 14, **characterized in that**,
the execution mechanism is a motion motor and or an energy generation apparatus of a laser knife, an ultrasound knife, a water jet cutter and or an electrotome.

16. A biplanar ultrasound image planning apparatus, which is a biplanar ultrasound image planning apparatus captured by an ultrasonic probe, using the method according to any one of claims 1 to 15, and comprising: an ultrasonic imaging module, a control module and a display module, **characterized in that**,

the ultrasonic imaging module is configured to acquire a position of a marker and generate a cross-sectional image and a sagittal image;
the control module is configured to:

establish a coordinate system of the marker according to the position of the marker, and respectively and correspondingly establish a coordinate system of the cross-sectional image and a coordinate system of the sagittal image according to the positions of the cross-sectional image and the sagittal image,
calculate a first conversion matrix converted from the coordinate system of the cross-sectional image to the coordinate system of the marker, and a second conversion matrix converted from the coordinate system of the sagittal image to the coordinate system of the marker, and
calculate a third conversion matrix and or a fourth conversion matrix based on the first conversion matrix and the second conversion matrix; and
the display module is configured to: through the third conversion matrix and or the fourth conversion matrix, display a target position in the other coordinate system in a follow-up manner according to the target position in either of the coordinate system of the cross-sectional image and the coordinate system of the sagittal image.

Calibrate a position relationship between a first ultrasonic probe and a cross-sectional image to obtain a first conversion matrix ~ 101

Calibrate a position relationship between the first ultrasonic probe and a sagittal image to obtain a second conversion matrix ~ 102

Calculate a third and/or fourth index conversion matrix by using a matrix conversion relationship, and display a target position displayed in any one of the coordinate system of the cross-sectional image and the coordinate system of the sagittal image in the other coordinate system in a follow-up manner ~ 103

FIG. 1(a)

Probe coordinate system

Image coordinate
system

FIG. 1(b)

FIG. 1(c)

Tracking coordinate system

$(0,0,0)$

$(R_1, T_1)$

$(R_2, T_2)$

$(X_2, Y_2, Z_2)$

$(X_1, Y_1, Z_1)$

Positioning and tracking sensor

$(R_0, T_0)$

$(R_0, T_0)$

$(X_0, Y_0, Z_0)$

Fixed position point

FIG. 1(d)

12 Coordinate system of
X positioning needle

Y

Z

13 X
Coordinate system
of positioning
needle point
Y

Z

FIG. 1(e)

Calibrate a position relationship between a first ultrasonic probe and a cross-sectional image to obtain a first conversion matrix — 101

Calibrate a position relationship between the first ultrasonic probe and a sagittal image to obtain a second conversion matrix — 102

Calculate a third or fourth conversion matrix by using a matrix conversion relationship — 104

Establish a first or second index relationship table in the movement process of the first ultrasonic probe — 105

Display a target position and a planned position in the cross-sectional image in a follow-up manner according to the target position and planned position in the sagittal image and by using the first or second index relationship table — 106

FIG. 2

Calibrate a position relationship between a first ultrasonic probe and a cross-sectional image to obtain a first conversion matrix 〜 101

Calibrate a position relationship between the first ultrasonic probe and a sagittal image to obtain a second conversion matrix 〜 102

Calculate a third or fourth conversion matrix by using a matrix conversion relationship 〜 104

Establish a third or fourth index relationship table in the movement process of the first ultrasonic probe 〜 107

Display a target position and a planned position in the sagittal image in a follow-up manner according to the target position and planned position in the cross-sectional image and by using the third or fourth index relationship table 〜 108

FIG. 3

Sagittal
ultrasound image Tn       Tx       T1

Sx

Sn      S......     S3 S2 S1

Cross-sectional
ultrasound image                        Ultrasonic target
detection object

Ultrasonic movement
direction indication

Ultrasonic probe    Ultrasonic probe adapter

Movement end position       Movement start position

Mn       Mx       M1

FIG. 4(a)

FIG. 4(b)

Calibrate a position relationship between a first ultrasonic probe and a cross-sectional image to obtain a first conversion matrix 〜 101

Calibrate a position relationship between the first ultrasonic probe and a sagittal image to obtain a second conversion matrix 〜 102

Calculate a third or fourth conversion matrix by using a matrix conversion relationship 〜 104

Establish a first or second index relationship table in the movement process of the first ultrasonic probe 〜 105

Display a target position and a planned position in the cross-sectional image in a follow-up manner according to the target position and planned position in the sagittal image and by using the first or second index relationship table 〜 106

Convert the planned position in the coordinate system of the cross-sectional image or the coordinate system of the sagittal image into a motion or energy execution parameter, where the motion or energy execution parameter is a parameter for controlling the motion and energy of an execution mechanism 〜 109

FIG. 5(a)

FIG. 5(b)

Calibrate a position relationship between a first ultrasonic probe and a cross-sectional image to obtain a first conversion matrix ～201

Calibrate a position relationship between a second ultrasonic probe and a sagittal image, then calibrate a position relationship between the second ultrasonic probe and the first ultrasonic probe, and obtain a second conversion matrix by using the matrix conversion relationship ～202

Calculate a third and/or fourth index conversion matrix by using a matrix conversion relationship, and display a target position displayed in any one of the coordinate system of the cross-sectional image and the coordinate system of the sagittal image in the other coordinate system in a follow-up manner ～203

Convert the planned position in the coordinate system of the cross-sectional image or the coordinate system of the sagittal image into a motion or energy execution parameter, where the motion or energy execution parameter is a parameter for controlling the motion and energy of an execution mechanism ～204

FIG. 6

FIG. 7(a)

FIG. 7(b)

FIG. 8

<table>
<tr><td colspan="2" align="center">**INTERNATIONAL SEARCH REPORT**</td><td>International application No.<br>**PCT/CN2023/083618**</td></tr>
</table>

**A. CLASSIFICATION OF SUBJECT MATTER**

A61B8/00(2006.01)i; A61B34/10(2016.01)i

According to International Patent Classification (IPC) or to both national classification and IPC

**B. FIELDS SEARCHED**

Minimum documentation searched (classification system followed by classification symbols)

IPC:A61B8,A61B34

Documentation searched other than minimum documentation to the extent that such documents are included in the fields searched

Electronic data base consulted during the international search (name of data base and, where practicable, search terms used)

CNABS, CNTXT, CNKI: 双平面, 双截面, 超声, 规划, 计划, 坐标, 转换, 转化, 矩阵, 位置, 标定; VEN, USTXT, WOTXT, EPTXT, IEEE, Elsevier Science, SpringerLink: Dual plane, biplane, double plane, ultrasound, ultrasonic, plan, layout, coordinate, conversion, transform, transition, matrix, position, location, demarcate

**C. DOCUMENTS CONSIDERED TO BE RELEVANT**

| Category* | Citation of document, with indication, where appropriate, of the relevant passages | Relevant to claim No. |
|---|---|---|
| PX | CN 114376610 A (BEIJING ZHIYU MEDICAL TECHNOLOGY CO., LTD.) 22 April 2022 (2022-04-22)<br>    claims 1-16, and description, paragraphs 31-210 | 1-16 |
| A | CN 113133832 A (SHENZHEN INSTITUTES OF ADVANCED TECHNOLOGY, CHINESE ACADEMY OF SCIENCES) 20 July 2021 (2021-07-20)<br>    description, paragraphs 64-99 | 1-16 |
| A | CN 106510760 A (SUZHOU GUOKE KANGCHENG MEDICAL TECHNOLOGY CO., LTD.) 22 March 2017 (2017-03-22)<br>    entire document | 1-16 |
| A | CN 110169823 A (ARIEMEDI TECHNOLOGY SHIJIAZHUANG CO., LTD.) 27 August 2019 (2019-08-27)<br>    entire document | 1-16 |
| A | US 5776067 A (HITACHI MEDICAL CORP.) 07 July 1998 (1998-07-07)<br>    entire document | 1-16 |

☑ Further documents are listed in the continuation of Box C.     ☑ See patent family annex.

| | |
|---|---|
| *   Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **29 June 2023** | **06 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

<div align="center">

**INTERNATIONAL SEARCH REPORT**

</div>

| International application No. |
| --- |
| **PCT/CN2023/083618** |

| C. | DOCUMENTS CONSIDERED TO BE RELEVANT | |
| --- | --- | --- |
| Category* | Citation of document, with indication, where appropriate, of the relevant passages | Relevant to claim No. |
| A | US 2012271162 A1 (SIEMENS CORP.) 25 October 2012 (2012-10-25)<br>entire document | 1-16 |
| A | US 2021321981 A1 (KONINKLIJKE PHILIPS N.V.) 21 October 2021 (2021-10-21)<br>entire document | 1-16 |

Form PCT/ISA/210 (second sheet) (July 2022)

**INTERNATIONAL SEARCH REPORT**
**Information on patent family members**

International application No.

**PCT/CN2023/083618**

| Patent document cited in search report | | | Publication date (day/month/year) | Patent family member(s) | | | Publication date (day/month/year) |
|---|---|---|---|---|---|---|---|
| CN | 114376610 | A | 22 April 2022 | CN | 114376610 | B | 10 June 2022 |
| CN | 113133832 | A | 20 July 2021 | CN | 113133832 | B | 20 September 2022 |
| | | | | WO | 2022198615 | A1 | 29 September 2022 |
| CN | 106510760 | A | 22 March 2017 | CN | 207444958 | U | 05 June 2018 |
| | | | | CN | 106510760 | B | 14 March 2023 |
| CN | 110169823 | A | 27 August 2019 | | None | | |
| US | 5776067 | A | 07 July 1998 | JPH | 09192131 | A | 29 July 1997 |
| | | | | JP | 3691895 | B2 | 07 September 2005 |
| US | 2012271162 | A1 | 25 October 2012 | US | 9271678 | B2 | 01 March 2016 |
| US | 2021321981 | A1 | 21 October 2021 | EP | 3613352 | A1 | 26 February 2020 |
| | | | | WO | 2020038835 | A1 | 27 February 2020 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210291564X